# EUROPÄISCHE PATENTSCHRIFT

(11) **EP 1 645 228 B1**
(45) Veröffentlichungstag und Bekanntmachung des Hinweises auf die Patenterteilung: **22.08.2007**
(21) Anmeldenummer: 04023719.0
(22) Anmeldetag: 05.10.2004
(51) Int. Cl.: A61B 5/06, A61B 19/00

(54) **Trackingsystem mit Streueffekt-Nutzung**
Tracking system using a scattering effect
Système de localisation utilisant un effet de dispersion

(43) Veröffentlichungstag der Anmeldung: 12.04.2006
(73) Patentinhaber: BrainLAB AG, 85551 Kirchheim/Heimstetten (DE)
(72) Erfinder: Goldbach, Günter, 85661 Forstinning (DE)
(74) Vertreter: Schwabe - Sandmair - Marx

(56) Entgegenhaltungen:
- WO-A1-97/47240
- WO-A1-20/04078039
- DE-A1- 19 639 615
- US-A1- 2003 225 329
- US-A1- 2004 122 311

## Beschreibung

Die Erfindung betrifft im Allgemeinen das technische Gebiet der Trackingsysteme, insbesondere der optischen und bevorzugt der medizinischen Trackingsysteme. Auf dem genannten technischen Gebiet beschäftigt sich die vorliegende Erfindung speziell mit einer Bilderzeugungsvorrichtung für ein optisches, insbesondere medizinisches Trackingsystem, mit dem die Position eines aufgenommenen Bildpunktes bestimmt wird. Ferner umfasst sie ein medizinisches Trackingsystem mit einer solchen Bilderzeugungsvorrichtung sowie ein Verfahren zur Bestimmung der Position eines aufgenommenen Bildpunktes.

Bekannte optische Trackingsysteme und deren Bilderzeugungsvorrichtungen umfassen meist ein Kamerapaar, welches einzelne Bildpunkte, meist Positionsmarker oder natürliche Landmarken, aufnimmt und aus dem stereoskopischen Bild Informationen über die Position, nämlich die räumliche Position der Bildpunkte, erfasst. Dabei gibt es Systeme, die im Kameraträger schon eine Bildverarbeitungseinheit aufweisen und als Ausgangssignal schon die räumlichen Koordinaten der erfassten Bildpunkte liefern. Grundsätzlich kann die Verarbeitung der Bilder aber auch an externer Stelle erfolgen.

Beispiele für allgemeine Trackingsysteme, welche eine optische 3D-Vermessung vornehmen, sind den folgenden Dokumenten zu entnehmen: DE 10 056 186 A1; US 5,831,735; US 6,493,095 B1; US 6,437,820 B1; US 5,828,770 und WO 00/16 121.

Auf dem medizinischen Gebiet werden optische Trackingsysteme beispielsweise in den folgenden Dokumenten vorgestellt: US 6,351,659 B1 oder DE 19 639 615 C2; US 6,484,049 B1 oder US 6,490475 B1 (fluoroskopisches Trackingsystem) und US 5,921,992.

Insofern sie Kamerasysteme mit einer vorgegebenen Bildauflösung verwenden, haben alle herkömmlichen Systeme den Nachteil, dass ihre Positionsbestimmungs-Genauigkeit wesentlich von der Auflösung der Bilderzeugungsvorrichtungen abhängig ist. Wenn relativ kleine Marker oder Landmarken aufgenommen und in ihrer Position bestimmt werden sollen oder bei Markern und Landmarken, die relativ weit von der Bilderzeugungsvorrichtung entfernt sind, stößt die Genauigkeit herkömmlicher Bilderzeugungsvorrichtungen schnell an ihre Grenzen, und ein Beispiel hierfür ist in der beiliegenden Figur 4 in der linken Bildreihe von oben nach unten erkennbar. Hier wird dargestellt, wie ein Bildpunkt P auf einem 8x8-Sensor einer herkömmlichen Bilderzeugungs-vorrichtung in der Position erfasst würde. Es ist zu bemerken, dass die Größe der Bildpunkte (z.B. eines Markers) auf dem Sensor im Sinne der Erfindung sehr viel kleiner sein kann als die Pixelgröße des Sensors, was in diesem herkömmlichen Fall nur zu einer maximalen Postionsgenauigkeit von einem Pixel führen kann.

In den drei linken Bildern der Figur 4 ist noch ein relativ guter Fall dargestellt, nämlich einer wo der Bildpunkt P vier Pixel des Sensors übergreift und damit in seiner Abbildung P' (mittleres Bild auf der linken Seite in Figur 4) 4 Pixel in unterschiedlicher Stärke belichtet. Eine Interpolation der "Helligkeit" (eigentlich der Entwicklungs- oder Ausleuchtungsstärke) der Pixelgruppe führt zu einer Sub-Pixel-Auflösung, welche den "Schwerpunkt" der Abbildung P' in etwa im Bereich des tatsächlichen Bildpunktes P ermittelt. Rechnerisch wird also die Position des Punktes P annähernd dort bestimmt, wo er als P" im unteren Bild der linken Seite in Figur 4 zu sehen ist.

Wie oben schon angemerkt, ist eine solche Sub-Pixel-Auflösung nur dann möglich, wenn der Bildpunkt P tatsächlich mehr als nur einen Pixel belichtet. Im schlechtesten Fall, wo der Bildpunkt P innerhalb eines einzigen Pixels liegt, kann seine Position nur als innerhalb des Pixels liegend festgestellt werden; bei kleinen Markern oder Bildpunkten kann hierbei ein großer Fehler entstehen.

Die Genauigkeit wird also durch die Auflösung des Sensors und die Dynamik und Linearität der Sensorpixel (und im 3D-Fall durch die Homogenität des Markers) bestimmt. Im Falle von medizinischen Trackingsystemen oder anderen Trackingsystemen, wo es in vielen Fällen auf Genauigkeiten von Bruchteilen von Millimetern ankommt, kann in der Regel ein Genauigkeitsfehler von einer Pixelgröße nicht toleriert werden, weshalb stets versucht wird, Marker möglichst groß zu machen, um die Position über die Mittelung von mehren belichteten Pixeln zu bestimmen..

Anders betrachtet werden die Größe von Markern und ihr maximaler Abstand zur Bilderzeugungsvorrichtung klar durch die Auflösung und den Reproduktions-Maßstab der Optiken bestimmt. Zwar könnte die Größe von Bildpunkten, wenn Marker verwendet werden, dadurch erhöht werden, dass man einfach größere Marker nimmt, was jedoch erhebliche Nachteile in der Handhabbarkeit, bei den Kosten und beim Gewicht der Marker mit sich bringt..

Ferner müssen für solche Bildpunkt-Positionsbestimmungen noch einige Voraussetzungen erfüllt werden, nämlich:
- die Belichtung der Pixel wird nur durch ein einziges Objekt ausgelöst, weil sonst die Gewichtung der Graustufen zur Subpixelauflösung fehlschlägt (diese Annahme ist kritisch);
- der Schwerpunkt ist das interpolierte Zentrum für die Helligkeit (dies ist nicht der Fall, wenn ein Marker teilweise verdeckt, nicht rund oder verschmutzt ist, oder die reflektierende Fläche nicht gleichmäßig hell abgebildet wird);
- ein runder Marker produziert dasselbe Bild auf zwei Sensoren, wenn man von zwei verschiedenen Winkeln auf ihn sieht (dies ist ebenfalls dann nicht der Fall, wenn der Marker nicht rund, verschmutzt oder teilweise verdeckt ist, oder die Helligkeit der reflektierenden Fläche nicht homogen ist.);
- das Bild des Markers ist groß genug, um mindestens einen Pixel zu überdecken (in der Praxis mindestens vier); und
- der Marker ist hell genug, um den Marker von dem Hintergrund-Rauschen unterscheiden zu können.

Alle diese Annahmen und Einschränkungen bringen Nachteile für die herkömmlichen Trackingsysteme mit sich. Man hat versucht, die Probleme dadurch zu lösen, dass Bilderzeugungsvorrichtungen mit immer höheren Auflösungen verwendet werden. Jedoch sind sehr hoch auflösende Bildsensoren auch sehr teuer und steigern damit die Kosten erheblich. Auch können einige der oben genannten Probleme nicht einfach dadurch gelöst werden, dass äußerst hoch auflösende Bilderzeugungsvorrichtungen verwendet werden, beispielsweise wenn Bildpunkte nicht vollständig sichtbar sind, z.B. bei verschmutzen Markern. Ein weiterer Nachteil ist, dass hochauflösendere Sensoren in der Regel eine deutlich kleinere Pixelfläche haben und damit unempfindlicher sind, was zu potentiell längeren Belichtungszeiten führt und damit bei bewegten Objekten kritisch ist.

Die Entwickler von Trackingsystemen befinden sich also in einer Art "Zwickmühle": Einerseits kann man die Marker nicht beliebig groß machen und andererseits kann man die Auflösung nicht beliebig erhöhen.

Aus der WO 97/47240 ist ein Positionsbestimmungssystem bekannt, bei dem Marker mit Lichtstreueinrichtungen versehen sind. Die WO 2004/078039 A1 beschreibt eine Instrumentenlokalisierung mit einem Katheter, der Lichtstreuabschnitte an Lichtleiterenden aufweist.

Es ist die Aufgabe der vorliegenden Erfindung, eine Bilderzeugungsvorrichtung bzw. ein optisches, insbesondere medizinisches Trackingsystem und ein Positions-Bestimmungsverfahren für ein solches System bereitzustellen, das den Weg aus der oben genannten "Zwickmühle" weist.

Diese Aufgabe wird durch eine Bilderzeugungsvorrichtung gelöst, wie sie gemäß dem Patentanspruch 1 definiert ist. Die erfindungsgemäße Bilderzeugungsvorrichtung für ein optisches, insbesondere medizinisches Trackingsystem, mit dem die Position eines aufgenommenen Bildpunktes bestimmt wird, weist eine Lichtstreueffekt-Erzeugungseinrichtung auf. Die vorliegende Erfindung geht damit einen völlig neuen Weg, der aus dem oben erwähnten Dilemma herausführt. Anstatt die Marker größer oder die Auflösung höher zu machen, wird die Lichtstreueffekt-Erzeugungseinrichtung verwendet, die das Bild eigentlich "unschärfer" macht. Mit der gerichteten, definierten Streuung des Lichtes eines Markers bietet sich die Möglichkeit, auf einem Sensor der Bilderzeugungsvorrichtung eine größere Anzahl von Pixeln zu belichten, wodurch eine räumlich größere Menge an Information auf dem Bildsensor zur Auswertung zur Verfügung steht. Vorteilhaft wirkt sich dabei aus, dass bei der Erzeugung von Lichtstreueffekten Helligkeitsgradienten entstehen, d.h., an der Stelle, wo der Bildpunkt tatsächlich liegt, wird er am hellsten abgebildet, und mit der Entfernung verringert sich diese Helligkeit. Dadurch steht ein Gradient zur Verfügung, der bei der Ermittlung der genauen Bildpunktposition verwendet werden kann.

Kurioserweise wird also die Bildpunktbestimmung genauer, obwohl durch den Lichtstreueffekt das gesamte Bild eigentlich unschärfer gemacht wird.

Mit der erfindungsgemäßen Bilderzeugungsvorrichtung kann deshalb ein Bildaufnahmesystem verwendet werden, das eine relativ geringe Auflösung hat. Die Marker müssen nicht mehr groß sein, sie können vorteilhafterweise sogar sehr klein sein, solange sie ausreichend Helligkeit abstrahlen oder reflektieren und damit determinierbare Bildpunkte und Streuungsmuster entstehen lassen. Die Erfindung gibt also ihre Anforderungen an die Schärfe des Bildes auf, indem sie die Detektion der Bildpunkte leichter macht. Die Information der tatsächlichen Bildpunktposition wird durch eine optimale Kombination von Bildauflösung und Dynamik (Empfindlichkeit) des Sensors und der Qualität des Streumusters erzielt. Falls bekannte und spezielle Lichtstreueffekte verwendet werden, wird die Gesamtenergie, die von dem Bildpunkt detektiert wird, über eine Anzahl von Pixeln in einem bekannten Muster und in bekannten Winkeln verteilt.

Die oben zuletzt genannte Verteilung wird bei der vorliegenden Erfindung realisiert, bei der die Lichtstreueffekt-Erzeugungseinrichtung eine Sterneffekt- bzw. Kreuzeffekt-Erzeugungseinrichtung ist, die Stern- bzw. Kreuzeffekte in zwei Richtungen erzeugt, die in einem vorbestimmten Winkel zueinander stehen, insbesondere im Winkel von 90°.

Es können auch Stern- bzw. Kreuzeffekte höherer Ordnung erzeugt werden, beispielsweise in mindestens drei Richtungen, die in einem vorbestimmten Winkel zueinander stehen, insbesondere in einem Winkel von 180° geteilt durch die Anzahl der Richtungen, also mit gleichem Winkelabständen.

Mit den obigen Ausführungsformen entsteht der besondere Vorteil gegenüber einem gezielten, diffusen Defokusieren, dass man durch die Ausgestaltung der Lichtstreueffekt-Erzeugungseinrichtung schon diejenigen Winkel vorgeben kann, in welchem das Kreuz- bzw. Sternmuster erzeugt werden wird. Mit solchen vorbekannten Richtungen lassen sich die Kreuzungspunkte und damit die Bildpunkte selbst sehr viel einfacher erfassen und ermitteln, da man lediglich noch die parallel zu den Sternstrahlen hellste Anordnung ermitteln muss, die dann eine Linie des "Fadenkreuzes" bildet. Am Kreuzungspunkt befindet sich dann die Abbildung des Bildpunktes, beispielsweise des Markers.

Es soll hier bemerkt werden, dass Sterneffekt- bzw. Kreuzeffekt-Erzeugungseinrichtungen nicht die einzigen vorstellbaren Lichtstreueffekt-Erzeugungseinrichtung im Rahmen der vorliegende Erfindung sind. Ebenfalls wäre es beispielsweise denkbar, verschiedenste Arten von Weichzeichnereffekten zu verwenden, um die Helligkeit von Bildpunkten beispielsweise gleichmäßig und homogen nach außen zu streuen, wobei sie graduell abnimmt.

Die Lichtstreueffekt-Erzeugungseinrichtung kann verschiedene Ausführungsformen annehmen. Eine Hardware-Konfiguration wäre beispielsweise ein Lichtstreueffekt-Filter, insbesondere ein Sterneffekt- bzw. Kreuzeffektfilter, der vor, hinter, auf oder in einem Kameralinsensystem angeordnet ist.

Die Erfindung umfasst ferner ein optisches, insbesondere medizinisches Trackingsystem mit einer Bilderzeugungsvorrichtung, wie sie oben beschrieben wurde. Ein solches Trackingsystem kann gemäß einer Ausführungsform eine Bildverarbeitungseinheit umfassen, die anhand des Lichtstreumusters, insbesondere anhand der Sterneffekt-Linien und/oder deren Kreuzungspunkten die Position eines Bildpunktes ermitteln.

Gemäß einer weiteren Ausführungsform umfasst ein solches Trackingsystem eine Bilderzeugungsvorrichtung mit mindestens zwei beabstandeten Kameras, sowie mit einer Bildverarbeitungseinheit, die anhand des Lichtstreumusters, nämlich der Sterneffekt-Linien und/oder deren Kreuzungspunkten die zweidimensionale Position eines Bildpunktes im Bild jeder Kamera ermittelt und aus den ermittelten zweidimensionalen Positionen eine räumliche Position des Bildpunktes errechnet.

Ferner betrifft die vorliegende Erfindung noch ein Verfahren zur Bestimmung der Position eines aufgenommenen Bildpunktes mittels eines optischen, insbesondere medizinischen Trackingsystems, bei dem
- mit einer Bilderzeugungsvorrichtung ein Bild erzeugt wird,
- auf dem Bild für vorbestimmte Bildpunkte ein Lichtstreueffekt, nämlich ein Sterneffekt- bzw. Kreuzeffekt erzeugt wird; und bei dem
- anhand des auf dem Bild abgebildeten Lichtstreumusters, nämlich anhand der Sterneffekt- bzw. Kreuzeffekt-Linien und/oder deren Kreuzungspunkten die Position eines Bildpunktes ermittelt wird.

Hierbei besteht die Möglichkeit, die Position des Bildpunktes unter Berücksichtigung der vorbekannten und vorbestimmten Winkel von Sterneffekt- bzw. Kreuzeffektlinien zueinander zu bestimmen. Die Stern- bzw. Kreuzeffekte können in zwei Richtungen erzeugt werden, die in einem vorbestimmten Winkel zueinander stehen, insbesondere im Winkel von 90°. Es besteht aber auch die Möglichkeit, die Effekte in mindestens drei Richtungen zu erzeugen, die in einem vorbestimmten Winkel zueinander stehen, insbesondere in einem Winkel von 180° geteilt durch die Anzahl der Richtungen.

In besonders vorteilhafter Ausführungsform werden die Stern- bzw. Kreuzeffekte in mindestens einer Anzahl von Richtungen erzeugt, welche die Anzahl der positionsmäßig zu ermittelnden Bildpunkte um 1 übersteigt. Mit dieser Konfiguration lässt sich ein Bildpunkt immer dort sicher ermitteln, wo sich die höchste Anzahl von Effektlinien kreuzen

Die Position des Bildpunktes kann erfindungsgemäß zusätzlich anhand des Helligkeits-Gradienten des Lichtstreumusters, insbesondere der Stern- bzw. Kreuzeffektlinien ermittelt werden, da dort wo der Bildpunkt abgebildet wird die größte abgebildete Helligkeit sein wird.

Die Erfindung wird nun im Weiteren anhand von Beispielen und Ausführungsformen näher erläutert. Sie kann alle hierin beschriebenen Merkmale einzeln sowie in jedweder Kombination aufweisen. In den beiliegenden Figuren, auf die hierbei Bezug genommen wird, zeigen:
- Figur 1 und Figur 2: Beispiele für Bilder mit und ohne Stern- bzw. Kreuzeffekte;
- Figur 3: Beispiele für die Bestimmung von Bildpunkten auf Kamerasensoren mit Hilfe von Stern- bzw. Kreuzeffekten;
- Figur 4: eine Gegenüberstellung der Bildpunkt-Positionsermittlung in herkömmlicher Weise und mit erfindungsgemäßen Lichtstreueffekten;
- Figur 5: eine schematische Darstellung eines erfindungsgemäßen optischen Trackingsystems;
- Figur 6: einen Vergleich der Größe herkömmlicher Reflektorenmarker mit Punktlicht-Markern;
- Figur 7: eine Ausführungsform einer Lichtleiteranordnung mit Lichtsammeleinrichtung;
- Figur 8: das Ende eines Lichtleiters mit einer Punktlichtquelle; und
- Figur 9: verschiedene Markerreflektoren mit Diamantschliff.

Ihre besondere Anwendung findet die vorliegende Erfindung im Rahmen der bildgeführten, navigationsgestützten medizinischen Behandlung, wo die Position eines Patienten oder eines medizinischen Instruments bzw. einer medizinischen Behandlungsapparatur mit Hilfe von Markern und mit Hilfe eines Kamerasystems (Trackingsystem) detektiert wird, um eine bildunterstützte Chirurgie durchführen zu können.

Erfindungsgemäß lassen sich insbesondere hierbei geringere Gesamtsystemkosten erzielen, es können extrem kleine und leichte Marker verwendet werden, die Marker-Erfassungsprozeduren werden vereinfacht, die Genauigkeit wird erhöht und die Markererfassung wird robuster und vertrauenswürdiger.

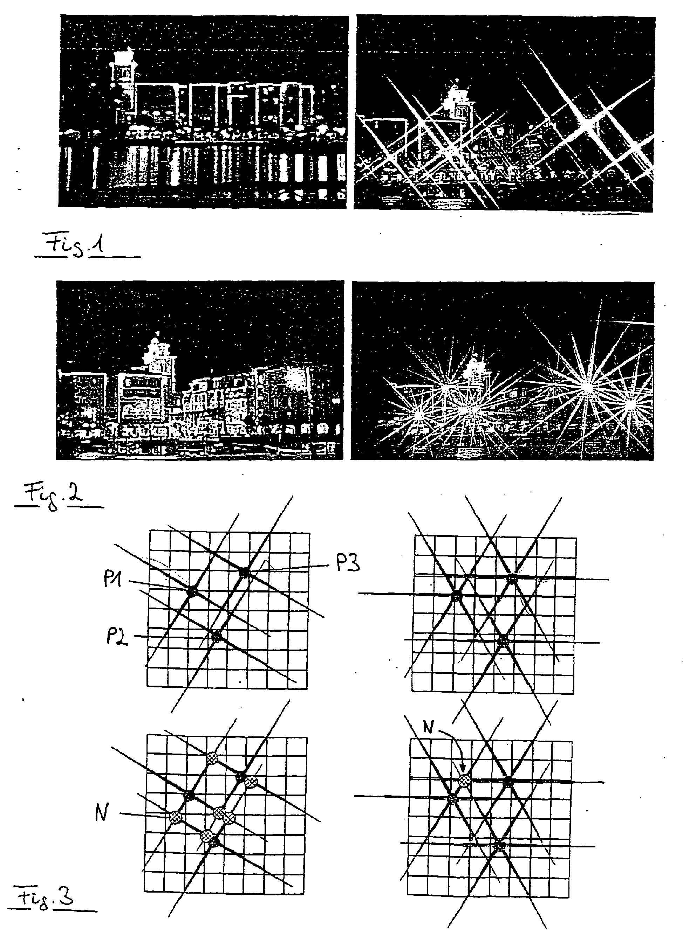

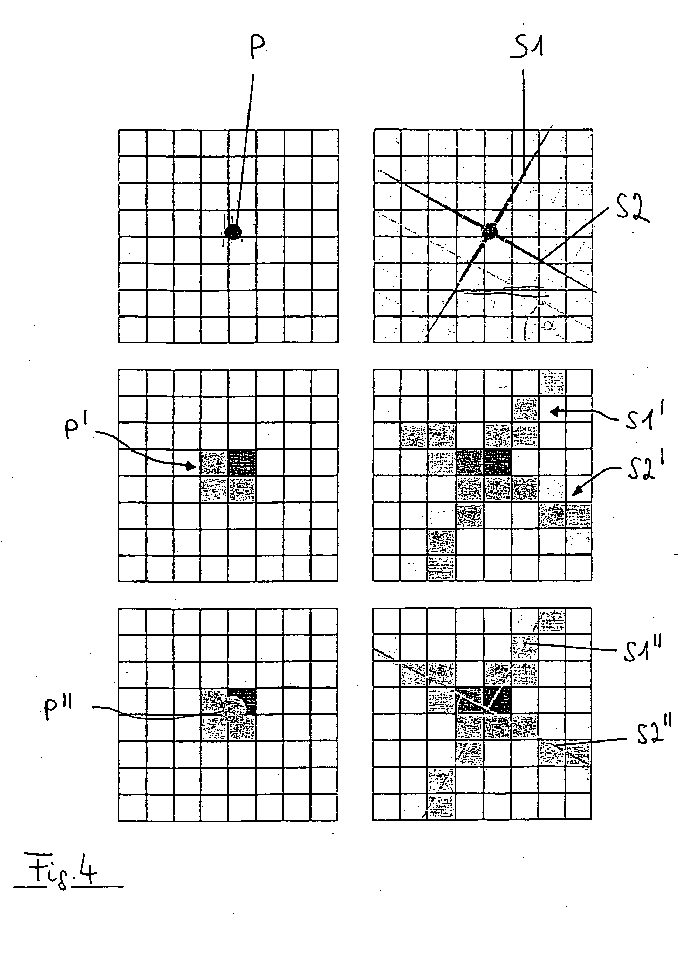

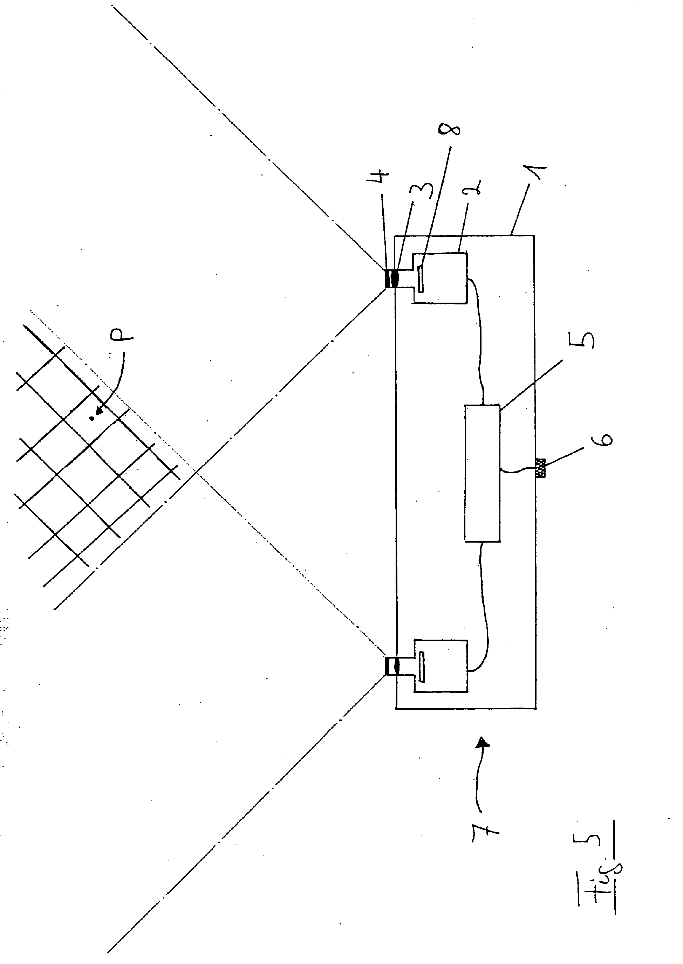

Lichtstreueffekte, nämlich im vorliegenden Beispiel Sterneffekte bzw. Kreuzeffekte, wie sie bei der vorliegenden Erfindung verwendet werden, werden beispielsweise in den Figuren 1 und 2 aufgezeigt. Die beiden gegenübergestellten Bilder in den Figuren 1 und 2 zeigen grundsätzlich den Effekt eines Stern- bzw. Kreuzfilters in einem Bild mit mehreren hellen Bildpunkten (Spotlights). Wie ersichtlich wird, ergibt sich bei der Verwendung eines solchen Effektes an den Spotlight ein Kreuzmuster (Figur 1) oder ein Sternmuster (Figur 2), je nach Effekt-Ordnung. Der Kreuzungspunkt der senkrechten Strahlen (Figur 1) ist das Zentrum des Bildpunktes, also des Spotlights, nämlich der hellste Punkt. Wenn Unklarheiten über potentielle Bildpunktpositionen auftreten könnten, weil sich die Strahlen verschiedener Bildpunkte kreuzen und damit mehrere Kreuzungspunkte entstehen, kann dieses Problem basierend auf der Helligkeit bzw. dem Helligkeitsgradienten der Strahlen gelöst werden, oder durch die Einbeziehung von Informationen über die Strahlenform sowie durch die Verwendung von Stern- bzw. Kreuzfiltern höherer Ordnung, wie beispielsweise in Figur 2 gezeigt ist.

Erzielt werden kann der Stern- bzw. Kreuzeffekt auf verschiedene Weise, wie Eingangs schon beschrieben wurde. Ein Beispiel ist die Verwendung eines Stern- oder Kreuzfilters, und wie dieser verwendet wird ist in Figur 5 zu sehen. Die Figur 5 zeigt die schematische Darstellung eines optischen, insbesondere eines medizinischen Trackingsystems 7. Dieses weist ein Gehäuse 1 auf, das mit zwei Kameras 2 bestückt ist. In der Figur 5 ist jedes Teil nur einmal mit einem Bezugszeichen versehen, auch wenn es, wie bei den Kameras, zweimal vorhanden ist.

Die Kameras 2 weisen einen gemeinsamen Sichtbereich auf, der kreuzschraffiert gezeichnet ist, und das Trackingsystem 7 kann, wenn es kalibriert ist, die dreidimensionale Position eines Bildpunktes in einem vorbestimmten Koordinatensystem ermitteln, wenn der Bildpunkt in dem gemeinsamen Sichtbereich liegt. Dazu geben die beiden Kameras 2 die zweidimensional ermittelte Position jeder Kamera an eine Bildverarbeitungseinheit 5 weiter, welche aus den beiden Informationen eine dreidimensionale Position ermittelt und diese über den Ausgang 6 ausgibt. An den Ausgang 6 kann beispielsweise ein medizinisches Navigationssystem angeschlossen werden, das dann eine bildgeführte Chirurgie ermöglicht, wenn mit dem Trackingsystem Marker an Patienten, Instrumenten oder medizinischen Apparaturen positionsmäßig ermittelt werden.

Bei der vorliegenden Ausführungsform weisen die Kameras 2 jeweils eine Linse 3 auf, die vor dem Bildsensor 8 liegt. Außen vor der Linse ist ein Stern- bzw. Kreuzeffekt-Filter aufgesetzt, der das Bezugszeichen 4 hat. Mit diesem Kamerasystem lässt sich nun also der Bildpunkt, beispielsweise der eingezeichnete Bildpunkt P positionsmäßig ermitteln, und im Weiteren wird zur Erläuterung wieder auf die Figur 4 Bezug genommen.. Der Stern- bzw. Kreuzfilter kann zur Optimierung des Aufbaus auch mit geeigneten Oberflächenbearbeitungstechniken direkt auf das Linsensystem aufgebracht werden, oder kann sich in einer weiteren Form im bzw. hinter dem Linsensystem befinden.

Einleitend wurde schon beschrieben, welche Schwierigkeiten bei der herkömmlichen Bildpunkt-Positionsbestimmung auftreten, und zwar anhand der drei links untereinander angeordneten Bilder in Figur 4. Die drei rechts untereinander angeordneten Bilder zeigen nun den Effekt eines 4-fach-Sternfilters bei der Positionsbestimmung des gleichen Bildpunktes P, beispielsweise eines Markers. Dabei werden durch den Stern- bzw. Kreuzfilter Strahlen S1 und S2 erzeugt, welche sich zunächst als eine Anzahl von Pixelbelichtungen S1', S2' auf dem Sensor abbilden. Nunmehr ist eine viel größere Anzahl von Pixeln auf dem Sensor belichtet und aus den unterschiedlichen Helligkeiten lassen sich mit sehr viel höherer Genauigkeit wiederum die Strahlen S1" und S2" ermitteln, deren Kreuzungspunkt exakt die Position des Markers bzw. des Bildpunktes wiedergibt.

Im Einzelnen wird klar, dass die Interpolation der Position der Strahlen S1, S2, basierend auf der Helligkeit der zugeordneten Pixel eine sehr viel präzisere Schätzung des Schwerpunktes des Objektes bereitstellt, und zwar basierend auf der Tatsache, dass die Ausrichtung der Strahlen einerseits als gerade (und in diesem Fall senkrecht zueinander) bekannt ist, und zusätzlich die Richtung der Strahlen bekannt ist, nämlich definiert durch die Ausgestaltung des Stern- bzw. Kreuzfilters. Eine Objekt-Detektion (Segmentierung) kann deshalb dadurch vereinfacht werden, dass Kreuzungspunkte gerader Linien gesucht werden, bei denen der Winkel der Linien schon durch Gestaltungsvorgaben bekannt ist. Die Gesamt-Helligkeit des Markers wird über viele Pixel verteilt, und im vorliegenden Fall resultiert sie in einem geometrischen Muster, wobei der Kreuzungspunkt der geraden Linie das Zentrum des Markers ist. Das Bild des Markers könnte merklich kleiner sein als die Größe eines Pixels, da die Optik den Strahl über viele Pixel verteilt.

Vorteilhafterweise verlässt sich die Prozedur nicht länger auf Algorithmen, die einen Marker-Umriss erfassen müssen und aus diesen Informationen den Mittelpunkt finden. Die Kanten müssen nicht mehr exakt detektiert werden, da der Mittelpunkt des Markers basierend auf den tatsächlichen Helligkeitsgradienten und der Form der belichteten Pixel und zusätzlich aus der geometrischen Kreuzungspunkt-Information der Strahlen errechnet werden kann.

Basierend auf dieser Prozedur kann die Markergröße merklich reduziert werden, so dass ein Marker praktisch ein Punkt mit vernachlässigbarer Größe werden kann, solange er hell genug abstrahlt. Das Problem ungleichmäßig ausgeleuchteter, schmutziger oder abgedeckter Marker wird somit praktisch eliminiert.

Die Prozedur basiert auf den folgenden Annahmen:
- die Lichtquelle, welche das Stern- bzw. Kreuzmuster erzeugt, ist hell. In der Praxis kann die Lichtquelle Infrarot- oder UV-Licht emittieren oder reflektieren, so dass keine Irritation des Bedieners auftritt und Normallicht die Erfassung nicht stört;
- die Lichtquelle, welche das Stern- bzw. Kreuzmuster erzeugt, kann klein sein, so dass der Schwerpunkt definitiv der Helligkeits-Mittelpunkt ist;
- eine Abdeckung, Anfeuchtung, oder Verschmutzung des Markers bzw. Größentoleranzen sind vernachlässigbar, da der Marker in Bezug auf solche potentiellen Störungen sehr klein ist. Wenn ein Marker abgedeckt ist, ist es besser, er liefert keinen Bildpunkt statt einen falschen Bildpunkt. Der fehlende Bildpunkt kann z.B. durch einen weiteren (redundanten) Marker zur Verfügung gestellt werden;
- das Bild des Markers kann sehr klein sein, solange es hell genug ist, um ein Kreuzmuster aus mehreren Pixeln zu erzeugen.

Die oben genannten Annahmen sind sehr viel weniger kritisch als die eingangs angesprochenen Annahmen für die herkömmliche Bildpunkt-Positionserfassung (Schwerpunkt Berechnung), bei der die Form die Markers eine große Rolle spielt.

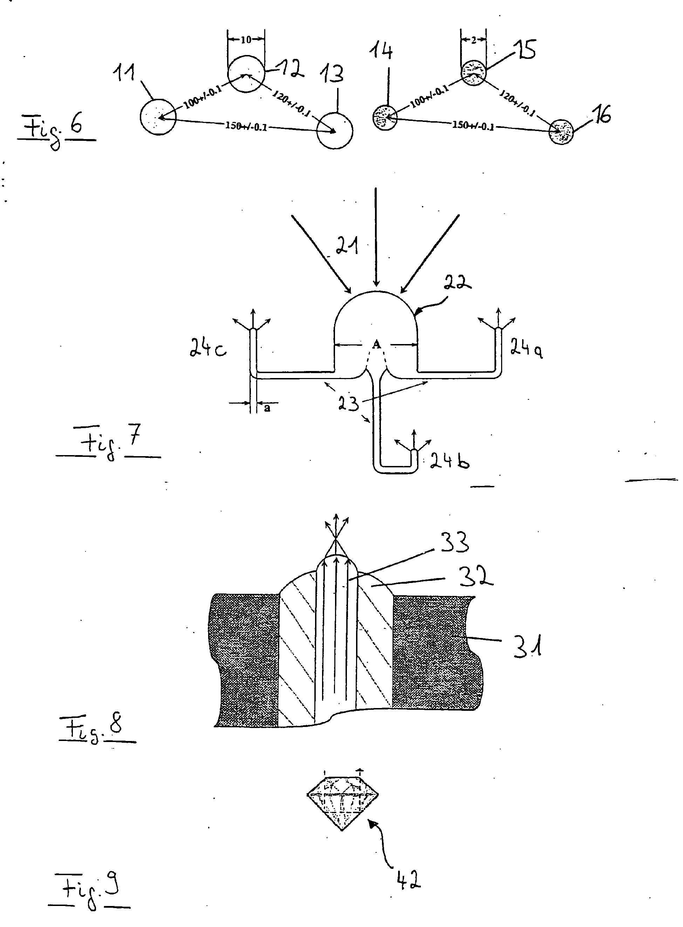

Oben wurde schon kurz eine Art Ausschlussverfahren angesprochen, mit dem verhindert wird, dass gemäß der vorliegenden Erfindung falsche Bildpunkte identifiziert werden. In Figur 3 ist ein solches Ausschlussverfahren nochmals dargestellt. Die beiden Darstellungen auf der linken Seite in Figur 3 zeigen oben die richtig identifizierten Bildpunkte P1, P2 und P3 an bestimmten Kreuzungspunkten mehrerer Lichtstrahlen. Im Bild darunter ist dargestellt, dass sich aber, wenn man lediglich die Kreuzungspunkte betrachtet, eigentlich mehrere Punkte ergeben würden, wobei einer als nicht echter Bildpunkt N bezeichnet ist. Die nicht echten Bildpunkte sind schraffiert gezeichnet.

Diese Situation könnte einerseits dadurch behoben werden, dass man Stern- bzw. Kreuzfilter mit höherer Ordnung verwendet, wie beispielsweise im Bild rechts oben in Figur 3. Mit dieser Maßnahme lässt sich dann sagen, dass nur dort, wo drei Strahlen sich kreuzen, tatsächlich ein Bildpunkt sein kann.

Auch in diesem Fall besteht noch die Möglichkeit, dass Unklarheiten bleiben. So ist im Bild rechts unten in Figur 3 gezeigt, dass der Punkt N ebenfalls der Kreuzungspunkt dreier Strahlen ist, obwohl er nicht tatsächlich einen Bildpunkt darstellt. In solchen Fällen hilft man sich, indem man die Helligkeit des Punktes n insgesamt zur Beurteilung heranzieht. Weil die Strahlen nach außen vom Zentrum weg an Helligkeit abnehmen wird der Punkt N nicht das gleiche Maß an Helligkeit aufweisen wie die tatsächlichen Bildpunkte, und er kann somit ausgeschlossen werden. Ein weiterer Hinweis für einen korrekten Marker ist die Tatsache, dass die Strahlen S1 uns S2 wie in Figur 8 rechts dargestellt am Kreuzungspunkt immer die gleiche Helligkeit haben. Dieses Kriterium ist für die Punkte N in Figur 7 nicht erfüllt. Die Ausschlussreihenfolge lautet also:
1. Kreuzungspunkt, 2. Kreuzungspunkt in der Ordnung des Stern- bzw. Kreuzfilters, 3. Kreuzungspunkt mit höchster Helligkeit und 4. Kreuzungspunkt mit gleicher Helligkeit der Strahlen.
Es lassen sich also in jedem Fall die richtigen Bildpunkte P1, P2 und P3 ermitteln.

Die vorliegende Erfindung eignet sich speziell zur Verwendung oder Kombination mit einem Positionsmarkersystem mit mindestens einem lichtabstrahlenden oder lichtreflektierenden Marker, der positionell von einem optischen, insbesondere medizinischen Trackingsystem erfassbar ist, wobei der effektive abstrahlende bzw. reflektierende Teil des oder der Marker als Punktlichtquelle bzw. Punktlichtreflektor ausgebildet ist.

Der Begriff "Punktlichtquelle" umfasst Lichtquellen mit sehr kleinen Abmessungen, deren Position genau auf einem Kamerasensor bestimmt werden kann und deren Helligkeit ausreicht, um den Sensor zu belichten.

Der oben genannte effektive abstrahlende bzw. reflektierende Teil des oder der Marker wird im Weiteren auch als "Punktlicht" bezeichnet. Diese Punktlichter sind insbesondere solche, die in Abstrahlrichtung ein homogenes Licht abstrahlen, also aus allen Richtungen ihres Leuchtbereiches dasselbe Bild abgeben.

Eine mögliche Definition der Größe der Punktlichter, also der Größe des effektiv abstrahlenden bzw. reflektierenden Teil des oder der Marker, ist eine Definition über ihre Positionierungstoleranz (Positionierungsgenauigkeit). Eine Positionierungstoleranz bzw. eine Positionierungsgenauigkeit ist auf dem Gebiet der optischen Trackingsysteme allgemein die Genauigkeit der Lagebestimmung von Markern im Raum (meist relativ zueinander, aber auch absolut). Dabei haben die Punktlichter bevorzugt eine Größe, die geringer ist als das 25-fache ihrer Positionierungstoleranz.

Die Punktlichter können eine Größe von höchstens 3 mm, insbesondere höchstens 2 mm und vorzugsweise höchstens 1 mm oder einen Bruchteil von 1 mm haben. Ferner können sie eine Größe von mindestens 30 µm haben, insbesondere mindestens 50 µm und vorzugsweise mindestens 62,5 µm.

Ein solches Positionsmarkersystem ist vorzugsweise so ausgestaltet, dass das Punktlicht oder die Punktlichter so bearbeitet oder ausgebildet ist bzw. sind, dass sie Licht in räumlich gleichmäßiger Verteilung abstrahlen. Bei einer Ausführungsform ist beispielsweise das Ende eines lichtleitenden Innenteils einer Lichtleiterfaser so poliert oder geschliffen, dass es eine konvexe Streulinse bildet.

Gemäß einer bevorzugten Ausführungsform des Positionsmarkersystems weist dieses mehrere Marker auf, welche die Enden von Lichtleitern sind, die aus einer gemeinsamen Lichtquelle gespeist werden, wobei die Lichtquelle eine aktiv abstrahlende Quelle oder eine Lichtsammeleinrichtung sein kann, die Umgebungslicht sammelt.

In anderer Ausführungsform können die Punktlichter Reflektorenmarker sein, die aus einem Material hergestellt sind, das einen bis zu 2,4 oder 2,6 hohen Brechungsindex aufweist, insbesondere aus einem Diamant-, Rutil-, Fabulit- oder Moissanit-Material.

Gemäß einer weiteren Ausführungsvariante sind das oder die Punktlichter Reflektorenmarker aus einem durchsichtigen oder halbdurchsichtigen Material, und sie weisen einen Kristall- oder Diamantschliff auf, insbesondere einen idealen Schliff.

Die Figur 6 zeigt eine Gegenüberstellung eines herkömmlichen (links) und eines Punktlicht-Positionsmarkersystems (rechts). Bei den dargestellten Markern handelt es sich um die tatsächlich reflektierende Fläche eines Reflektorenmarkers 11, 12, 13. In der Regel werden heute kugel- oder scheibenförmige retroreflektiemede Oberflächen verwendet, die über einen relativ großen Bereich Licht in die Ausgangsrichtung zurückwerfen, um ein möglichst großes Abbild des Markers im Trackingsystem zu erzeugen. Drei solcher Marker können beispielsweise an einem Patienten-Körperteil oder auf einem festen Körper (Markergeometrie oder englisch rigid body) angebracht werden, um dessen Position im Raum mittels eines Trackingsystems zu ermitteln. Die Marker 11, 12 und 13 des herkömmlichen Markersystems weisen untereinander Abstände von 100, 120 bzw. 150 mm auf, und sie haben einen Durchmesser von 10 mm. Die Positionierungstoleranz bzw. die Positionierungsgenauigkeit, also die Genauigkeit der Lagebestimmung von einzelnen Markern im Raum (relativ zueinander, aber auch absolut) ist hier mit +/- 0,1 mm angegeben. Dies ist eine übliche Positionierungstoleranz.

Die Marker 14, 15, 16 des in Figur 1 rechts dargestellten Punktlicht-Positionsmarkersystems sind sehr viel kleiner ausgestaltet, wobei die tatsächlich reflektierende Fläche hier nochmals wesentlich kleiner sein kann oder sogar soll.

Retroreflektierende Oberflächen zur Vergrößerung der effektiven Markerfläche werden deshalb nicht benötigt. Im vorliegenden Fall haben die Marker bei gleicher Positionierungstoleranz und gleichen Abständen zueinander nur einen Durchmesser von 2 mm, und dies entspricht in etwa dem 20-fachen der Positionierungstoleranz.

Die Verwendung von Punktlichtquellen-Markern führt zu präziseren zweidimensionalen Positionsmessungen (mit einer Kamera), erhöht aber auch die Genauigkeit bei einer dreidimensionalen Erfassung, wenn ein stereoskopisches Kamerasystem verwendet wird. Dies liegt einerseits daran, dass ein Punktlichtquellen-Marker mit größerer Wahrscheinlichkeit aus zwei Richtungen dasselbe Bild abgibt als ein relativ großer Marker, da mechanische Deformationen, Abdeckung sowie eine ungleichmäßige Helligkeitsverteilung mit einfachen Mitteln eliminiert werden können und die Positionsinterpolation nicht länger beeinflussen. Die als einzelne leuchtende Objekte ausgestalteten Punktlichtquellen-Marker haben eine Reihe von Vorteilen. Der potentielle Fehler bei der Berechnung oder Abschätzung des Mittelpunktes (Schwerpunktes) ist prinzipiell kleiner. Das Risiko einer teilweisen Verdeckung von Markern und deshalb einer nicht korrekten Abschätzung des Schwerpunktes ist begrenzt, weil die Marker entweder ganz abgedeckt werden oder überhaupt nicht. Wenn ein Marker ganz abgedeckt wird, muss dies nicht zu einem Ausfall des Systems führen, da grundsätzlich mehr als die benötigte Marker-Mindestanzahl zum Orten eines einzigen Objektes bereitgestellt werden können und weil nach einer gewissen Zeit ein Marker nicht mehr abgedeckt sein wird, so dass die Ortung korrekt weitergehen kann. Bei medizinischen Anwendungen, beispielsweise beim Tracking von chirurgischen Instrumenten wird die zu erwartende Größe von abdeckenden Objekten (Blut, Wassertropfen, Finger eines Chirurgen, etc.) typischerweise größer sein als die des Punktlicht-Markers. Dadurch ist sichergestellt, dass nicht ein nur teilweise sichtbarer Marker positionell falsch detektiert wird, was die Positionsbestimmung insgesamt verfälschen würde. Weitere Vorteile betreffen das geringere Gewicht von Instrumenten, die mit solchermaßen kleinen Markern versehen sind und die Möglichkeit, Marker in sehr dünne Instrumente integrieren zu können, beispielsweise in Katheter, Bohrwerkzeuge, etc.

Das Punktlicht-Positionsmarkersystem kann ein aktives oder passives Markersystem sein, also Punktlichtquellen aufweisen, die selbst Licht abstrahlen oder lediglich Licht reflektieren. Ferner können aktive Systeme, also mit selbst abstrahlenden Markern, unterschiedliche Lichtversorgungen aufweisen. Es können Lichtquellen bereitgestellt werden, die ihre eigene Energieversorgung aufweisen, also beispielsweise eine Lichtquelle, die von einer Batterie, einem Akku, einer Fuel Cell, oder einer Solarzelle gespeist wird, die oder der in einem kabellosen Instrument vorgesehen wird, welches mittels der Punktlichtquellen geortet werden soll. Natürlich kann auch von außen ein Lichtleiterkabel an die Positionsmarker-Lichtversorgung angeschlossen werden oder eine externe Energieversorgung für eine Lichtquelle in dem Objekt, das mit dem Markern versehen wird.

Eine besonders vorteilhafte Ausführungsform ist eine solche, bei der ein kabelloses Objekt, also beispielsweise ein chirurgisches Instrument, bereitgestellt wird, das ohne eigene oder äußere (elektrische) Energieversorgung arbeiten kann und trotzdem eine sehr hohe Energiedichte der Positionsmarker-Punktlichter aufweist. Ein Positionsmarkersystem für ein solches chirurgisches Instrument (oder für chirurgische Behandlungsgeräte bzw. zur Markierung von Patienten-Körperteilen) weist eine Lichtsammeleinrichtung auf, von der sich mindestens ein Lichtleiter erstreckt, dessen Ende eine Punktlichtquelle bildet. In Figur 7 ist schematisch eine solche Anordnung gezeigt. Außenlicht 21, nämlich Umgebungslicht oder Licht aus einer aktiven Lichtquelle (ständig leuchtende oder intermittierende Lichtquelle) wird in einer relativ großen Kollimatorlinse 22 gesammelt. Im Falle medizinischer Anwendungen kann diese Lichtsammellinse 22 beispielsweise am hinteren Ende eines Instruments positioniert sein. Sie kann gut durch sehr helle Operationslichter mit sichtbarem Licht versorgt werden oder durch eine separate Lichtquelle die unsichtbares Licht bzw. unsichtbare elektromagnetische Strahlung (ultraviolett oder infrarot) emittiert. Die Kollimatorlinse 22 ist so aufgebaut, dass sie sich in eine Anzahl kleiner Lichtleiter 23 aufspaltet, die das von der Linse 22 gesammelte Licht zu den präzise positionierten Punktlichtquellen 24a bis c transportieren. Die Lichtleiter 23 können aus einem flexiblen oder steifen Kunststoffmaterial oder Glasmaterial hergestellt sein, mit optimierten Lichttransmissionseigenschaften, wie dies aus optischen Glasfaser-Datenübertragungsleitungen bekannt ist. Die Helligkeit des an den Enden bzw. Punktlichtquellen 24a bis c abgestrahlten Lichtes ist eine Funktion der Größe (Maß a) der Kollimatorlinse 22 in Bezug auf die Apertur der Punktlichtquelle 24a bis c (Maß a), die Anzahl der verwendeten Lichtleiter und in Bezug auf die optischen Eigenschaften des verwendeten Materials.

Basierend auf diesem Prinzip kann ein relativ heller Punktlichtmarker mit passiver Beleuchtung erzielt werden. Je kleiner die Apertur (a) ist, desto höher ist die Lichtdichte an den Enden 24a bis 24c. Stärkeres Umgebungslicht resultiert in einer höheren Marker-Helligkeit und stellt deshalb ein gutes Signal/Rauschen-Verhältnis über einen weiten Bereich der Eigenschaften des einfallenden Lichtes zur Verfügung.

Wie oben bereits angedeutet, besteht eine Möglichkeit zur Herstellung von aktiven Punktlichtmarkern mit sehr geringer Größe darin, herkömmlich erhältliche optische Faserkabel entweder aus Glas oder Kunststoff zu verwenden, wie sie beispielsweise in der Telekommunikation genutzt werden. Der lichtleitende innere Teil einer Glasfaseranordnung hat typischerweise eine Größe von 50 bis 62,5 µm für MultiMode-Fasern. Optische Kunststofffasern sind mit Durchmessern bis zu einigen Millimetern erhältlich. Es ist bekannt, wie man aus einem Laser oder einer LED-Lichtquelle intensives Licht in eine solche Faser einbringt.

Die Figur 8 zeigt eine Möglichkeit der Ausgestaltung einer Punktlichtquelle mit einem Lichtleiter. Der Lichtleiter 33 selbst ist üblicherweise mit einer Umhüllung 32 umgeben, die wiederum in dem Objekt verankert ist, das mit der Punktlichtquelle versehen werden soll, beispielsweise in der Wandung 31 eines chirurgischen Instruments. Der Lichtleiter 33 selbst tritt an seinem Ende etwas aus seiner Umhüllung 32 hervor. Dieses Ende ist bei der dargestellten Ausführungsform poliert oder geschliffen und bildet somit eine konvexe Verteilerlinse, die das Licht gleichmäßig in alle Abstrahlrichtungen abgibt.

Eine andere Art, das Licht am Ende der Faser gleichmäßig zu verteilen besteht beispielsweise darin, das Ende der Glas- oder Kunststofffaser durch Implantation oder Aufbringung von kleinen Partikeln in der Oberfläche streuend zu machen, oder durch eine gleichmäßige Anbringung von Vertiefungen oder Rillen auf der Faser-Stirnseite.

Eine andere Ausführungsform besteht in der Bereitstellung von passiven Punktlichtreflektoren. Der Vorteil solcher Punktlichtreflektoren liegt ganz allgemein darin, dass sie keine Versorgung mit Licht oder Energie benötigen und deshalb die bereitzustellenden Strukturen sehr viel einfacher werden. Es werden Punktlichtreflektoren bereitgestellt, die einen hohen Grad an Rückreflektion liefern können, und dies wird beispielsweise durch die Verwendung von Materialien möglich, die einen hohen Brechungsindex aufweisen, wie z.B. Diamant-, Rutil-, Fabulit- oder Moissanit-Materialien, welche einen Brechungsindex in der Größenordnung von 2,4 bis 2,6 aufweisen. Der Effekt ist als "Diamanteffekt" bekannt. Bei Materialien mit hohem Brechungsindex liegt der kritische Winkel für die Totalreflektion (innere Totalreflektion) in der Größenordnung von 24°. Dies bedeutet, dass das einfallende Licht mit einem extrem breiten Bereich an Beleuchtungswinkeln reflektiert wird.

Die Verwendung spezieller geometrischer Formen für die Kristalle ist ebenfalls möglich, um einfallendes Licht aus einer anderen als der Sichtrichtung zu verwenden, um einen hellen Lichteffekt (Spotlight-Effekt) zu erzeugen. Dies kann das Setup für eine Anzahl von Anwendungen stark vereinfachen, wo Positionsmessungen eingeschränkt werden durch Kamerasysteme mit geringer Größe, geringem Gewicht und einfacher Handhabung. Der Marker 42 in Figur 9 zeigt den klassischen Diamantenschliff, wie er verwendet werden kann, um stark rückstrahlende Effekte zu erzeugen.

Es können nunmehr sehr kleine Marker (Kristalle, Diamanten) in der Größenordnung eines Millimeter-Bruchteils, verwendet werden, und deshalb ist es nicht mehr notwendig, große Kristalle oder Anordnungen aus vielen Kristallen zur Anwendung zu bringen. Solche stark rückstrahlenden, passiven Punktlichtquellen-Marker ermöglichen eine starke Kostenreduktion, sie sind fast gewichtslos und können in alle möglichen, auch sehr kleine zu ortenden Objekte eingebracht werden.

Eine weitere Ausführungsform der Punklichtquellen-Marker stellen polierte oder vergütete Metall- oder Kunstoffkugeln dar, die entgegen gängiger Markersysteme klein sein können (siehe Fig. 1, rechts). Vorteil der Verwendung von Metall oder Kunstoffkugeln ist die Adaption der neuen Markertechnologie auf eine Vielzahl von bereits vorhandenen (chirurgischen) Instrumenten. Eine kostengünstige Massenproduktion sowie eine erhöhte Haltbarkeit der Marker gegenüber gängigen Markern (insbesondere gegenüber retroreflektierenden Oberflächen) sind weitere Vorteile.

## Patentansprüche

1. Bilderzeugungsvorrichtung für ein optisches, insbesondere medizinisches Trackingsystem (7), mit dem die Position eines aufgenommenen Bildpunktes (P) bestimmt wird, wobei die Bilderzeugungsvorrichtung eine Lichtstreueffekt-Erzeugungseinrichtung (4) aufweist, **dadurch gekennzeichnet, dass** die Lichtstreueffekt-Erzeugungseinrichtung eine Sterneffekt- bzw. Kreuzeffekt-Erzeugungseinrichtung (4) ist, die Stern- bzw. Kreuzeffekte in zwei ausgeprägten Richtungen erzeugt, die in einem vorbestimmten Winkel zueinander stehen, insbesondere im Winkel von 90°.

2. Bilderzeugungsvomchtung nach Anspruch 1, **dadurch gekennzeichnet, dass** die die Lichtstreueffekt-Erzeugungseinrichtung eine Sterneffekt- bzw. Kreuzeffekt-Erzeugungseinrichtung (4) ist, die Stern- bzw. Kreuzeffekte in mindestens drei Richtungen erzeugt, die in einem vorbestimmten Winkel zueinander stehen, insbesondere in einem Winkel von 180° geteilt durch die Anzahl der Richtungen.

3. Bilderzeugungsvorrichtung nach einem der Ansprüche 1 oder 2, **dadurch gekennzeichnet, dass** sie mindestens eine Kamera (2) mit einem Lichtstreueffekt-Filter (4), insbesondere einem Sterneffekt- bzw. Kreuzeffektfilter (4) aufweist, der vor oder hinter der Kameralinse (3) oder auf dieser angeordnet ist.

4. Optisches, insbesondere medizinisches Trackingsystem mit einer Bilderzeugungsvorrichtung nach einem der Ansprüche 1 bis 3.

5. Trackingsystem nach Anspruch 4, **dadurch gekennzeichnet, dass** es eine Bildverarbeitungseinheit umfasst, die anhand des Lichtstreumusters, nämlich anhand der Sterneffekt-Linien und/oder deren Kreuzungspunkten die Position eines Bildpunktes ermittelt.

6. Trackingsystem nach Anspruch 4 oder 5, **dadurch gekennzeichnet, dass** es eine Bilderzeugungsvorrichtung mit mindestens zwei beabstandeten Kameras (2) umfasst, sowie eine Bildverarbeitungseinheit (5), die anhand des Lichtstreumusters, insbesondere der Sterneffekt-Linien und/oder deren Kreuzungspunkten die zweidimensionale Position eines Bildpunktes im Bild jeder Kamera (2) ermittelt und aus den ermittelten zweidimensionalen Positionen eine räumliche Position des Bildpunktes errechnet.

7. Verfahren zur Bestimmung der Position eines aufgenommenen Bildpunktes mittels eines optischen, insbesondere medizinischen Trackingsystems, bei dem
- mit einer Bilderzeugungsvorrichtung ein Bild erzeugt wird,
- auf dem Bild für vorbestimmte Bildpunkte (P) ein Lichtstreueffekt, nämlich ein Sterneffekt- bzw. Kreuzeffekt erzeugt wird; und bei dem
- anhand des auf dem Bild abgebildeten Lichtstreumusters, nämlich anhand der Sterneffekt- bzw. Kreuzeffekt-Linien und/oder deren Kreuzungspunkten die Position eines Bildpunktes (P) ermittelt wird.

8. Verfahren nach Anspruch 7, bei dem die Position des Bildpunktes unter Berücksichtigung der vorbekannten und vorbestimmten Winkel von Sterneffekt- bzw. Kreuzeffekt-Linien zueinander bestimmt werden.

9. Verfahren nach Anspruch 7 oder 8, bei dem Stern- bzw. Kreuzeffekte in zwei Richtungen erzeugt werden, die in einem vorbestimmten Winkel zueinander stehen, insbesondere im Winkel von 90°.

10. Verfahren nach Anspruch 7 oder 8, bei dem Stern- bzw. Kreuzeffekte in mindestens drei Richtungen erzeugt werden, die in einem vorbestimmten Winkel zueinander stehen, insbesondere in einem Winkel von 180° geteilt durch die Anzahl der Richtungen.

11. Verfahren nach einem der Ansprüche 7 bis 10, bei dem Stern- bzw. Kreuzeffekte in mindestens einer Anzahl von Richtungen erzeugt werden, welche die Anzahl der positionsmäßig zu ermittelnden Bildpunkte um 1 übersteigt.

12. Verfahren nach einem der Ansprüche 7 bis 11, bei dem die Position des Bildpunktes zusätzlich anhand des Helligkeits-Gradienten des Lichtstreumusters, insbesondere der Stern- bzw. Kreuzeffektlinien ermittelt wird.

13. Verfahren nach einem der Ansprüche 7 bis 11, bei dem die Position des Bildpunktes zusätzlich anhand der gleichen Helligkeit der Linien des Lichtstreumusters im Schnittpunkt bestimmt wird.

## Claims

1. An image generating device for an optical, in particular medical tracking system (7), using which the position of a recorded image point (P) is determined, wherein the image generating device comprises a light scattering effect generating means (4), **characterised in that** the light scattering effect generating means is a star and/or cross effect generating means (4) which generates star and/or cross effects in two distinct directions which are at a predetermined angle, in particular an angle of 90°, with respect to each other.

2. The image generating device according to claim 1, **characterised in that** the light scattering effect generating means is a star and/or cross effect generating means (4) which generates star and/or cross effects in at least three directions which are at a predetermined angle with respect to each other, in particular an angle of 180° divided by the number of directions.

3. The image generating device according to any one of claims 1 or 2, **characterised in that** it comprises at least one camera (2) including a light scattering effect filter (4), in particular a star and/or cross effect filter (4), which is arranged in front of, behind or on the camera lens (3).

4. An optical, in particular medical tracking system comprising an image generating device according to any one of claims 1 to 3.

5. The tracking system according to claim 4, **characterised in that** it comprises an image processing unit which ascertains the position of an image point on the basis of the light scattering pattern, namely on the basis of the star effect lines and/or their intersection points.

6. The tracking system according to claim 4 or 5, **characterised in that** it comprises an image generating device including at least two spaced cameras (2), and an image processing unit (5) which ascertains the two-dimensional position of an image point in the image of each camera (2) on the basis of the light scattering pattern, in particular the star effect lines and/or their intersection points, and calculates a spatial position of the image point from the ascertained two-dimensional positions.

7. A method for determining the position of a recorded image point by means of an optical, in particular medical tracking system, in which:
- an image is generated using an image generating device;
- a light scattering effect, namely a star and/or cross effect, is generated on the image for predetermined image points (P); and in which
- the position of an image point (P) is ascertained on the basis of the light scattering pattern projected on the image, namely on the basis of the star and/or cross effect lines and/or their intersection points.

8. The method according to claim 7, wherein the position of the image point is determined by taking into account the prior known and predetermined angles of star and/or cross effect lines with respect to each other.

9. The method according to claim 7 or 8, wherein star and/or cross effects are generated in two directions which are at a predetermined angle with respect to each other, in particular an angle of 90°.

10. The method according to claim 7 or 8, wherein star and/or cross effects are generated in at least three directions which are at a predetermined angle with respect to each other, in particular an angle of 180° divided by the number of directions.

11. The method according to any one of claims 7 to 10, wherein star and/or cross effects are generated in at least a number of directions which exceeds the number of image points to be positionally ascertained by 1.

12. The method according to any one of claims 7 to 11, wherein the position of the image point is additionally ascertained on the basis of the brightness gradient of the light scattering pattern, in particular the star and/or cross effect lines.

13. The method according to any one of claims 7 to 11, wherein the position of the image point is additionally determined on the basis of the same brightness of the lines of the light scattering pattern at the intersection point.

## Revendications

1. Dispositif générateur d'image pour un système de localisation (7) optique, en particulier médical, par lequel la position d'un point (P) d'une image prise est déterminée, le dispositif générateur d'image comportant un dispositif générateur (4) à effets de lumière diffuse, **caractérisé en ce que** le dispositif générateur à effets de lumière diffuse est un dispositif générateur d'effets à étoiles ou croix, qui produit des effets à étoiles ou croix dans deux directions marquées qui forment un angle prédéterminé l'une par rapport à l'autre, en particulier un angle de 90°.

2. Dispositif générateur d'image selon la revendication 1, **caractérisé en ce que** le dispositif générateur à effets de lumière diffuse est un dispositif générateur (4) à effets à étoiles ou croix qui produit des effets à étoiles ou croix dans deux directions marquées qui forment un angle prédéterminé l'une par rapport à l'autre, en particulier un angle de 180° divisé par le nombre de directions.

3. Dispositif générateur d'image selon l'une des revendications 1 ou 2, **caractérisé en ce qu'**il comporte au moins une caméra (2) avec un filtre à effets de lumière diffuse (4), en particulier un filtre à effets à étoiles ou croix (4) qui est disposé devant ou derrière la lentille (3) de la caméra ou sur celle-ci.

4. Système de localisation optique, en particulier médical comportant un dispositif générateur d'image selon l'une des revendications 1 à 3.

5. Système de localisation selon la revendication 4, **caractérisé en ce qu'**il comprend une unité de traitement d'image qui à l'aide du modèle de lumière diffuse, à savoir à l'aide des lignes à effets à étoiles et/ou leurs points de croisement, détermine la position d'un point d'image.

6. Système de localisation selon la revendication 4 ou 5, **caractérisé en ce qu'**il comprend un dispositif générateur d'image avec au moins deux caméras (2) espacées, ainsi qu'une unité de traitement d'image (5) qui, à l'aide du modèle de lumière diffuse, en particulier des lignes à effets à étoiles et/ou de leurs points de croisement, détermine la position bidimensionnelle d'un point d'image dans l'image de chaque caméra (2), et calcule, à partir des positions bidimensionnelles déterminées, une position spatiale du point d'image.

7. Procédé pour déterminer la position d'un point d'une image prise au moyen d'un système de localisation optique, en particulier médical, dans lequel
- une image est produite avec un dispositif générateur d'image,
- sur l'image, un effet de lumière diffuse, à savoir un effet à étoiles ou croix, est produit pour des points d'image (P) prédéterminés ; et dans lequel
- à l'aide du modèle de lumière diffuse reproduit sur l'image, à savoir à l'aide des lignes d'effets à étoiles ou croix et/ou de leurs points de croisement, on détermine la position d'un point d'image (P).

8. Procédé selon la revendication 7, dans lequel la position du point d'image est déterminée en tenant compte des angles connus et prédéterminés de lignes à effets à étoiles ou croix les unes par rapport aux autres.

9. Procédé selon la revendication 7 ou 8, dans lequel des effets à étoiles ou croix sont produits dans deux directions qui forment entre elles un angle prédéterminé, en particulier un angle de 90°.

10. Procédé selon la revendication 7 ou 8, dans lequel des effets à étoiles ou croix sont produits dans deux directions qui forment entre elles un angle prédéterminé, en particulier un angle de 180° divisé par le nombre de directions.

11. Procédé selon l'une des revendications 7 à 10, dans lequel des effets à étoiles ou croix sont produits dans au moins un nombre de directions qui dépasse de 1 le nombre de points d'image à déterminer quant à leur position.

12. Procédé selon l'une des revendications 7 à 11, dans lequel la position du point d'image est déterminée en supplément à l'aide du gradient de luminosité du modèle de lumière diffuse, en particulier des lignes à effets à étoiles ou croix.

13. Procédé selon l'une des revendications 7 à 11, dans lequel la position du point d'image est déterminée en supplément à l'aide de la même luminosité des lignes du modèle de lumière diffuse, au point d'intersection.
